# EUROPEAN PATENT SPECIFICATION

(11) **EP 3 950 020 B1**
(45) Date of publication and mention of the grant of the patent: **22.04.2026**
(21) Application number: 20783958.0
(22) Date of filing: 26.02.2020
(51) Int. Cl.: A61L 101/36, B01D 46/42, A61L 2/20, A61L 2/18, A61L 9/015, A61L 9/12, A61L 2/22

(54) **DECONTAMINATION DEVICES AND SYSTEM**
DEKONTAMINATIONSVORRICHTUNGEN UND SYSTEM
DISPOSITIFS ET SYSTÈME DE DÉCONTAMINATION

(30) Priority: 04.04.2019 JP 2019071711
(43) Date of publication of application: 09.02.2022
(73) Proprietor: Nitta Corporation, Osaka-shi, Osaka 556-0022 (JP)
(72) Inventor: IKEDA, Takuji, Yamatokoriyama-shi, Nara 639-1085 (JP); SHIGETA, Makoto, Yamatokoriyama-shi, Nara 639-1085 (JP)
(74) Representative: Winter, Brandl - Partnerschaft mbB
(86) International application number: PCT/JP2020/007742
(87) International publication number: WO 2020/202920

(56) References cited:
- EP-A1- 2 974 748
- WO-A1-03/104561
- JP-A- 2002 540 815
- JP-A- 2014 508 579
- JP-A- 2016 022 144
- JP-A- 2016 022 144
- JP-A- 2018 050 483
- JP-A- S60 220 067
- JP-B1- 6 476 378

## Description

### Technical Field

The present invention relates to a decontamination device and a system.

### Background Art

Patent Literature 1 discloses a decontamination device for decontaminating the inside of a biosafety cabinet. This decontamination device atomizes a peracetic acid disinfectant and further gasifies the resulting peracetic acid disinfectant, and circulates the gasified peracetic acid in the biosafety cabinet. Accordingly, a HEPA (High Efficiency Particulate Air) filter disposed in the biosafety cabinet is decontaminated (see Patent Literature 1).

Patent literature 2 describes a paper impregnated with hydrogen peroxide and acetic acid, and reaction between the hydrogen peroxide and acetic acid creates peracetic acid. The gaseous peracetic acid diffuses out of pores in the paper, creating a no-growth zone on the surface.

### Citation List

### Patent Literature

Patent Literature 1: JP 2016-22144A
Patent literature 2: WO 03/104561 A1

### Summary of Invention

### Technical Problem

However, the peracetic acid disinfectant is temporarily atomized in the decontamination device disclosed in Patent Literature 1 above, and thus not only gaseous peracetic acid but also mist-like peracetic acid may circulate in the biosafety cabinet depending on the temperature and humidity inside the biosafety cabinet. If mist-like peracetic acid is circulated, an air filter for particle removal (e.g., a HEPA filter) disposed in the biosafety cabinet may become wet, for example. If the air filter for particle removal become wet and dust collected by the air filter for particle removal absorbs moisture, when the air filter dries, the dust may be solidified in a state in which the dust forms a film between fibers. As a result, a pressure loss of the air filter may increase.

The present invention was made in order to resolve such issues, and it is an object thereof to provide a decontamination device and a system that can inhibit an air filter for particle removal from becoming wet when the air filter is decontaminated by peracetic acid.

### Solution to Problem

The present invention is defined in the appended claims. A decontamination device according to an aspect of the present invention is configured to decontaminate an air filter for particle removal. This decontamination device is configured to release gas containing peracetic acid such that the gas reaches the air filter and not release mist containing peracetic acid. The decontamination device includes a porous member impregnated with a chemical solution containing peracetic acid and a housing, which is provided with a fluid flow channel therein and an opening portion formed on the downstream side of the fluid flow channel. A blower is located at the flow channel and the porous member is disposed at the flow channel

According to this decontamination device, gas containing peracetic acid is released toward the air filter and mist is not released, and thus it is possible to further reduce the likelihood of the air filter becoming wet, compared to a case where mist containing peracetic acid is released. That is, according to this decontamination device, the likelihood of the air filter becoming wet is reduced, and thus it is possible to reduce the likelihood of dust collected by the air filter for particle removal being solidified in a state in which the dust forms a film between fibers and a pressure loss of the air filter is increased.

In the decontamination device, the gas may be generated without heating a chemical agent containing peracetic acid.

According to this decontamination device, it is possible to suppress decomposition of peracetic acid because the chemical agent containing peracetic acid is not heated. Also, according to this decontamination device, the temperature of the chemical agent containing peracetic acid is kept at about the same temperature as the temperature inside the space where the air filter to be decontaminated is disposed, and thus it is possible to reduce the likelihood of dew condensation occurring in the space.

The decontamination device may include a peracetic acid gas generation portion configured to store a chemical solution containing peracetic acid, and if the chemical solution is stored in the peracetic acid gas generation portion, the surface area of the chemical solution may be 20 cm² or more.

This decontamination device need only generate peracetic acid gas from the chemical solution containing peracetic acid. A method for generating peracetic acid gas may be a method in which the chemical solution is introduced into a container having a sufficiently wide opening so as to generate peracetic acid gas. Also, in a method for diffusing peracetic acid gas, gas may be diffused through natural diffusion or air may be blown by a blower, and for example, air may be diffused by a blower provided in a sealed container.

The decontamination device includes a porous member impregnated with the chemical solution containing peracetic acid.

The decontamination device may be configured to generate gas from the porous member impregnated with the chemical agent containing peracetic acid. In the method for diffusing peracetic acid gas, gas may be diffused through natural diffusion or air may be blown by a blower, and for example, air may be diffused by a blower provided in the sealed container.

The decontamination device further includes: a housing provided with a fluid flow channel therein and an opening portion formed on the downstream side of the fluid flow channel; and a blower disposed on the flow channel; in which the porous member is disposed on the flow channel.

With this decontamination device, as a result of the blower blowing air toward the porous member impregnated with the chemical agent, gas containing peracetic acid is released from the porous member toward the opening portion. Therefore, according to this decontamination device, gas containing peracetic acid is released toward the air filter, and thus it is possible to further reduce the likelihood of the air filter becoming wet, compared to a case where mist containing peracetic acid is released.

A decontamination device according to a further aspect of the present invention is configured to decontaminate an air filter for particle removal. This decontamination device is configured to release gas containing peracetic acid such that the gas reaches the air filter and not release mist containing peracetic acid. The decontamination device includes: a storage body configured to store the chemical solution containing peracetic acid; and a blower configured to blow air to the gas generated through evaporation of peracetic acid stored in the storage body; in which the gas to which the air has been blown by the blower may reach the air filter.

With this decontamination device, gas is released as a result of the blower blowing air toward the gas containing peracetic acid. Therefore, according to this decontamination device, gas containing peracetic acid is released toward the air filter, and thus it is possible to further reduce the likelihood of the air filter becoming wet, compared to a case where mist containing peracetic acid is released.

A decontamination device according to a further aspect of the present invention is configured to decontaminate an air filter for particle removal. This decontamination device is configured to release gas containing peracetic acid such that the gas reaches the air filter and not release mist containing peracetic acid. The decontamination device includes: a housing provided with a fluid flow channel therein and an opening portion formed on the downstream side of the fluid flow channel; a mist generator that is disposed on the flow channel and is configured to generate mist containing peracetic acid; a mist adsorption filter that is disposed on the downstream side of the flow channel relative to the mist generator and is configured to adsorb the mist; and a blower disposed on the flow channel.

With this decontamination device, mist generated in the mist generator is removed through a mist removal filter, and gas containing peracetic acid is released from the opening portion. Therefore, according to this decontamination device, gas containing peracetic acid is released toward the air filter, and thus it is possible to further reduce the likelihood of the air filter becoming wet, compared to a case where mist containing peracetic acid is released toward the air filter.

A system according to another aspect of the present invention includes a container and a decontamination device. The container is configured to hold an air filter for particle removal. The decontamination device is configured to release gas containing peracetic acid into the container such that the gas reaches the air filter and not release mist containing peracetic acid into the container.

According to this system, gas containing peracetic acid is released by the decontamination device toward the air filter in the container, and thus it is possible to further reduce the likelihood of the air filter becoming wet, compared to a case where mist containing peracetic acid is released.

### Advantageous Effects of Invention

According to the present invention, it is possible to provide a decontamination device that can inhibit a fibrous air filter from becoming wet when the air filter is decontaminated by peracetic acid.

### Brief Description of Drawings

FIG. 1 is a diagram illustrating an overview of a system.
FIG. 2 is a schematic diagram showing a front view of a decontamination device according to Embodiment 1.
FIG. 3 is a schematic diagram showing a cross-section taken along III-III in FIG. 2.
FIG. 4 is a diagram showing a state of the system during decontamination.
FIG. 5 is a flowchart showing a procedure of decontamination in the system.
FIG. 6 is a schematic diagram showing a front view of a decontamination device according to Embodiment 2.
FIG. 7 is a schematic diagram showing a cross-section taken along VII-VII in FIG. 6.
FIG. 8 is a diagram showing a schematic configuration of a decontamination device according to Embodiment 3.
FIG. 9 is a schematic diagram showing an experimental device in Experiment 1.
FIG. 10 is a schematic diagram showing an experimental device in Experiment 2.
FIG. 11 is a schematic diagram showing an experimental environment in Experiment 3.

### Description of Embodiments

Hereinafter, embodiments of the present invention will be described in detail with reference to the drawings. Note that, in the drawings, identical or corresponding portions have been assigned the same reference numerals, and their explanation is not repeated.

### 1. Embodiment 1

### 1-1. Overview of system

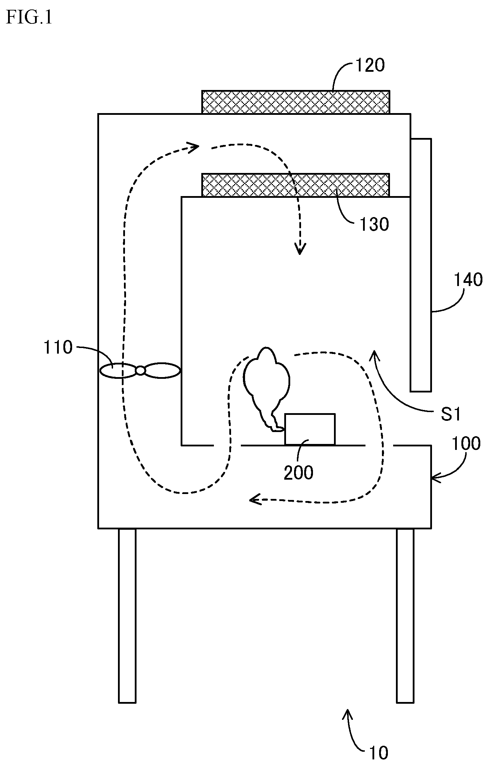

FIG. 1 is a diagram illustrating an overview of a system 10 according to this embodiment. As shown in FIG. 1, the system 10 includes a safety cabinet 100 and a decontamination device 200.

The safety cabinet 100 is a box-shaped experimental facility for suppressing biohazards. An experimenter inserts his/her hands into a workspace S1 and conducts experiments using a biological material, for example. The pressure of the workspace S1 is kept at negative pressure in the safety cabinet 100. That is, in the safety cabinet 100, the diffusion of a biological material disposed in the workspace S1 toward the experimenter is suppressed, for example.

The safety cabinet 100 includes a fan 110, HEPA (High Efficiency Particulate Air) filters 120 and 130, and a shutter 140. During an experiment, the operation of the fan 110 creates an air flow, and clean air is discharged to the outside via the HEPA filter 120, and clean air is supplied to the workspace S1 via the HEPA filter 130. The shutter 140 is configured to be openable and closable.

The decontamination device 200 is a device for decontaminating (bio-decontaminating) the HEPA filters 120 and 130 disposed in the safety cabinet 100 after the safety cabinet 100 has been used. The decontamination device 200 is disposed in the workspace S1 after the safety cabinet 100 has been used, for example. The decontamination device 200 is configured to decontaminate the HEPA filters 120 and 130 by releasing gaseous peracetic acid into the workspace S1. The reasons why the decontamination device 200 releases gaseous peracetic acid for decontaminating the HEPA filters 120 and 130 will be described below.

If mist-like peracetic acid is released by the decontamination device 200, the HEPA filters 120 and 130 may become wet depending on the humidity inside the safety cabinet 100. If the HEPA filters 120 and 130 become wet and the dust collected by the fibrous HEPA filters 120 and 130 absorbs moisture, the dust may solidify in a state in which the dust forms a film between fibers when the HEPA filters 120 and 130 are dry. As a result, the pressure loss of the HEPA filters 120 and 130 may increase. If the pressure loss increases, the HEPA filters 120 and 130 may burst, for example.

In view of this, with the system 10, the decontamination device 200 releases only gaseous peracetic acid into the workspace S1, thereby decontaminating the HEPA filters 120 and 130. According to the decontamination device 200, only the gas containing peracetic acid is released toward the HEPA filters 120 and 130, and thus it is possible to further reduce the likelihood of the HEPA filters 120 and 130 becoming wet, compared to a case where mist containing peracetic acid is released. That is, according to this decontamination device 200, the HEPA filters 120 and 130 are less likely to become wet, and thus it is possible to reduce the likelihood of the dust collected by the fibrous HEPA filters 120 and 130 solidifying in a state in which the dust forms a film between fibers and the pressure loss of the HEPA filters 120 and 130 is increased.

The following describes a configuration of the decontamination device 200 in detail, and describes a decontamination procedure performed in the safety cabinet 100.

### 1-2. Configuration of decontamination device

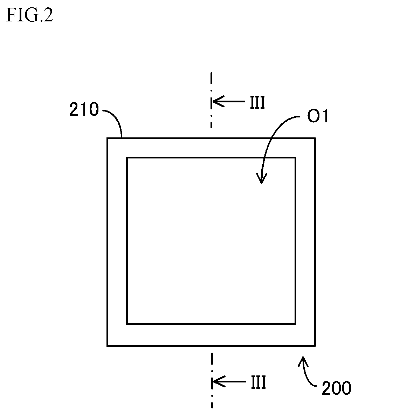

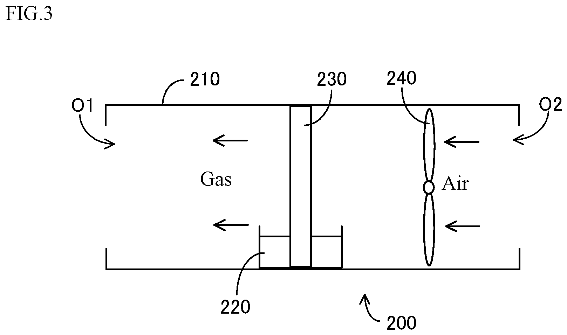

FIG. 2 is a schematic diagram showing a front view of the decontamination device 200. FIG. 3 is a schematic diagram showing a cross-section taken along III-III in FIG. 2. As shown in FIGS. 2 and 3, the decontamination device 200 includes a housing 210, a tray 220, a porous member 230, and a fan 240.

The housing 210 has a quadrangular prism shape in which a cavity (fluid flow channel) is formed inside the housing 210. The housing 210 is composed of resin or metal, for example. An opening portion O1 is formed in a front surface of the housing 210, and an opening portion O2 is formed in a back surface of the housing 210. The tray 220, the porous member 230, and the fan 240 are disposed on the flow channel formed inside the housing 210.

The tray 220 is configured to store a liquid chemical agent containing peracetic acid (also referred to as a "peracetic acid-based decontamination agent" hereinafter).

The porous member 230 need only be made wet due to the liquid chemical agent (chemical solution) containing peracetic acid. In this embodiment, the porous member 230 is disposed in the tray 220 that stores the chemical solution, and thus the porous member 230 is made wet due to the chemical solution. Note that a method for making the porous member 230 wet is not limited thereto, and the porous member 230 may be made wet by the chemical solution by applying the chemical solution to the porous member 230 from above the porous member 230, for example.

Note that the structure and members of the porous member 230 are not particularly limited as long as the porous member 230 is made wet by the liquid chemical agent containing peracetic acid and the porous member 230 can efficiently gasify the chemical agent by allowing air to pass therethrough. The porous member 230 may be formed by processing a sheet-shaped member such as a woven fabric, a knitted fabric, a non-woven fabric, or a film into a pleat shape or a corrugated shape, for example. A woven fabric, a knitted fabric, a non-woven fabric, a film, or the like may contain a porous material such as diatomaceous earth or zeolite. The porous member 230 is disposed in the tray 220. The porous member 230 sucks up the peracetic acid-based decontamination agent in the tray 220 through capillary action. That is, the porous member 230 is impregnated with the peracetic acid-based decontamination agent.

The fan 240 is configured to generate air that flows from the upstream side to the downstream side. Various known fans (blowers) can be adopted as the fan 240. The fan 240 is disposed on the upstream side relative to the porous member 230. That is, when the fan 240 operates, air that flows to the porous member 230 is generated, and only the gas containing peracetic acid is released from the porous member 230 toward the opening portion O1.

### 1-3. Decontamination procedure

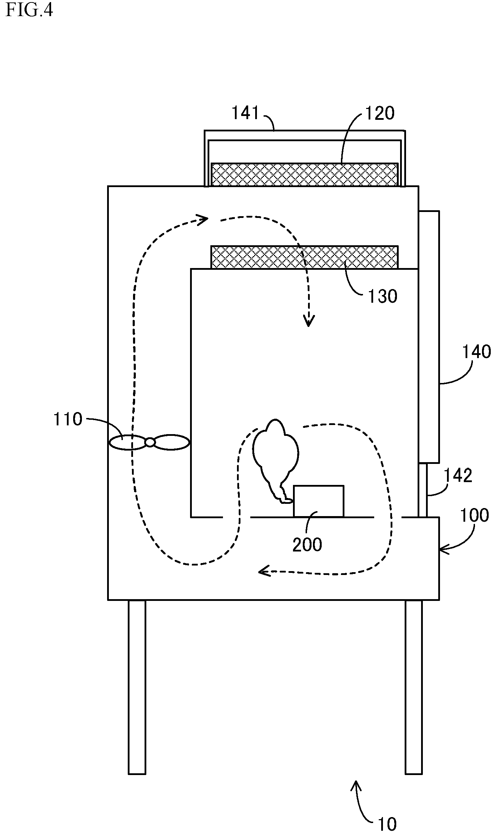

FIG. 4 is a diagram showing a state of the system 10 during decontamination. As shown in FIG. 4, during the decontamination of the system 10, the inside of the safety cabinet 100 is sealed or semi-sealed by seal members 141 and 142. Accordingly, leakage of peracetic acid gas to the outside of the safety cabinet 100 is suppressed, and thus the gas concentration in the safety cabinet 100 can be increased. Also, it is known that, through experimentation, even if sealing is realized by the seal member 141, the peracetic acid gas concentration above the HEPA filter 120 is sufficiently increased during decontamination.

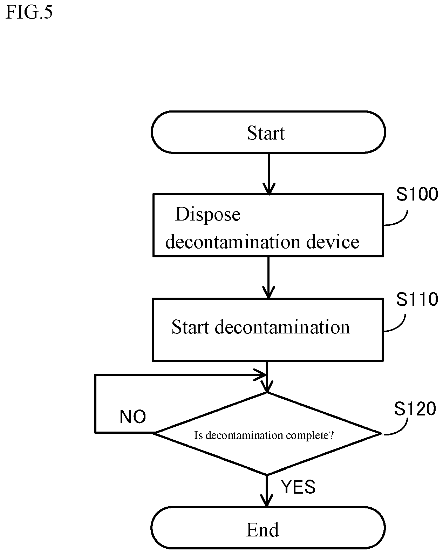

FIG. 5 is a flowchart showing a procedure of decontamination in the system 10. The processing shown in this flowchart is performed by an experimenter after the experimenter has used the safety cabinet 100, for example.

Referring to FIG. 5, the experimenter disposes the decontamination device 200 in the workspace S1 of the safety cabinet 100 (step S100). The experimenter causes the decontamination device 200 to start the decontamination of the HEPA filters 120 and 130 by operating the decontamination device 200 (step S110). Note that decontamination performed by the decontamination device 200 may be automatically started according to a timer setting or a setting made by a decontamination program, or may be started based on operation of a switch attached to a main body of the decontamination device or a switch provided outside the workspace S1.

The experimenter determines whether or not decontamination is complete (step S120), and if it is determined that decontamination has been completed (YES in step S120), the experimenter takes out the decontamination device 200 from the workspace S1 and completes the processing.

### 1-4. Characteristics

As described above, according to the decontamination device 200, only the gas containing peracetic acid is released toward the HEPA filters 120 and 130, and thus it is possible to further reduce the likelihood of the HEPA filters 120 and 130 becoming wet, compared to a case where mist containing peracetic acid is released. That is, according to this decontamination device 200, the HEPA filters 120 and 130 are less likely to become wet, and thus it is possible to reduce the likelihood of the dust collected by the fibrous HEPA filters 120 and 130 solidifying in a state in which the dust forms a film between fibers and the pressure loss of the HEPA filters 120 and 130 is increased.

In particular, in the decontamination device 200, the fan 240 blows air toward the porous member 230, and thus only the gas containing peracetic acid is released from the porous member 230 toward the opening portion O1. Therefore, according to the decontamination device 200, only the gas containing peracetic acid is released toward the HEPA filters 120 and 130, and thus it is possible to further reduce the likelihood of the HEPA filters 120 and 130 becoming wet, compared to a case where mist containing peracetic acid is released.

### 2. Embodiment 2

In Embodiment 1 described above, gaseous peracetic acid is released by the decontamination device 200. In this Embodiment 2, gaseous peracetic acid is released by a decontamination device 200A. The following mainly describes portions that are different from those in Embodiment 1.

### 2-1. Configuration of decontamination device

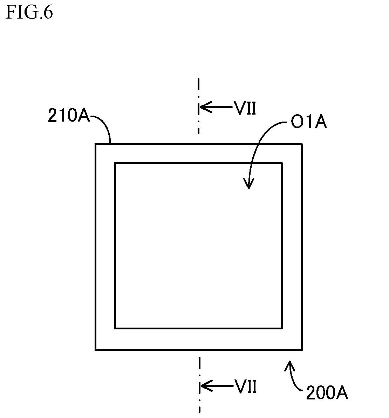

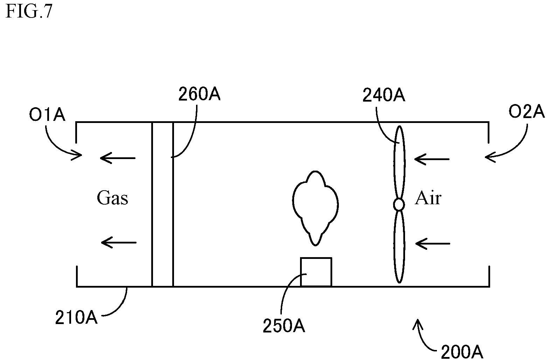

FIG. 6 is a schematic diagram showing a front view of the decontamination device 200A. FIG. 7 is a schematic diagram showing a cross-section taken along VII-VII in FIG. 6. As shown in FIGS. 6 and 7, the decontamination device 200A includes a housing 210A, a fan 240A, a mist generation device 250A, and a mist adsorption filter 260A.

The housing 210A has a quadrangular prism shape in which a cavity (fluid flow channel) is formed inside the housing 210A. The housing 210A is composed of resin or metal, for example. An opening portion O1A is formed in a front surface of the housing 210A, and an opening portion O2A is formed in a back surface of the housing 210A. The fan 240A, the mist generation device 250A, and the mist adsorption filter 260A are disposed on the flow channel formed in the housing 210A.

The fan 240A is configured to generate air that flows from the upstream side to the downstream side. Various known fans (blowers) can be adopted as the fan 240A.

The mist generation device 250A is configured to generate mist-like peracetic acid using a peracetic acid-based decontamination agent. Examples of a method for generating mist include an ultrasonic atomization method and a spraying method, and either one of them may be used. The mist generation device 250A is disposed on the downstream side relative to the fan 240A. That is, when the fan 240A and the mist generation device 250A operate, air carrying mist-like peracetic acid, which is generated by the mist generation device 250A, flows toward the mist adsorption filter 260A.

The mist adsorption filter 260A is disposed on the downstream side relative to the mist generation device 250A, and is configured to adsorb mist, which is generated by the mist generation device 250A. That is, in the decontamination device 200A, mist generated by the mist generation device 250A is adsorbed and vaporized by the mist adsorption filter 260A, and only the gas containing peracetic acid is released from the opening portion O1A.

### 2-2. Characteristics

As described above, according to the decontamination device 200A, only the gas containing peracetic acid is released toward the HEPA filters 120 and 130, and thus it is possible to further reduce the likelihood of the HEPA filters 120 and 130 becoming wet, compared to a case where mist containing peracetic acid is released. That is, according to this decontamination device 200A, the HEPA filters 120 and 130 are less likely to become wet, and thus it is possible to reduce the likelihood of the dust collected by the HEPA filters 120 and 130 for particle removal solidifying in a state in which the dust forms a film between fibers and the pressure loss of the HEPA filters 120 and 130 is increased.

In particular, in the decontamination device 200A, mist generated by the mist generation device 250A is removed by the mist adsorption filter 260A, and only the gas containing peracetic acid is released from the opening portion O1A. Therefore, according to the decontamination device 200A, only the gas containing peracetic acid is released toward the HEPA filters 120 and 130, and thus it is possible to further reduce the likelihood of the HEPA filters 120 and 130 becoming wet, compared to a case where mist containing peracetic acid is released to the HEPA filters 120 and 130.

It is more preferable that the mist adsorption filter 260A can adsorb mist and vaporize the adsorbed mist. Examples of the mist adsorption filter 260A include a filter obtained by forming a sheet-shaped member such as a woven fabric, a knitted fabric, or a non-woven fabric into a pleat shape. Examples of the material of the mist adsorption filter 260A include glass, resin, and cellulose, and resin and cellulose that have strength and resistance to bursting are preferable. In terms of the performance of the mist adsorption filter 260A, ULPA (Ultra Low Penetration Air) filters, HEPA filters, and medium efficiency penetration air filters are conceivable, and medium efficiency penetration air filters are more preferable in consideration of an improvement in the initial pressure loss and a pressure loss due to mist adhesion.

Note that, if an ultrasonic method is adopted as the method for generating mist, the decontamination agent may be heated by a vibrator because the decontamination agent is atomized as a result of the vibrator vibrating. When the decontamination agent is heated, the decomposition of peracetic acid is facilitated. Also, if the decontamination agent is heated, the temperature inside the safety cabinet 100 increases, and the difference between the room temperature and the temperature inside the safety cabinet 100 increases, the difference therebetween causing dew condensation.

### 3. Embodiment 3

In Embodiments 1 and 2 described above, gaseous peracetic acid is released respectively by the decontamination devices 200 and 200A. In this Embodiment 3, gaseous peracetic acid is released by a decontamination device 200B. The following mainly describes portions that are different from those in Embodiments 1 and 2.

### 3-1. Configuration of decontamination device

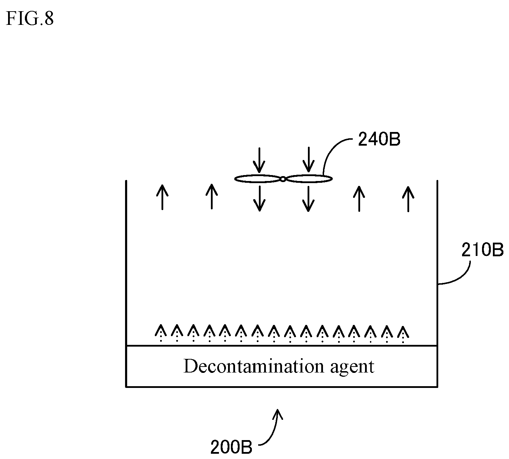

FIG. 8 is a diagram showing a schematic configuration of the decontamination device 200B according to Embodiment 3. As shown in FIG. 8, the decontamination device 200B includes a housing 210B and a fan 240B.

The housing 210B is composed of a material such as metal or resin that is not corroded by a decontamination agent, and is configured to store a decontamination agent 112. The decontamination agent is an aqueous solution containing peracetic acid. A fan 240B is provided above the housing 210B.

The fan 240B is configured to generate an air flow that flows from the outside toward the inside of the housing 210B and an air flow that flows from the inside toward the outside of the housing 210B, through rotation. Due to the rotation of the fan 240B, peracetic acid gas generated through evaporation of the decontamination agent is released to the outside of the housing 210B. In the decontamination device 200B, only the peracetic acid gas, which is generated through the evaporation of the decontamination agent, is released to the outside of the housing 210B, and thus no mist containing peracetic acid is released.

### 3-2. Characteristics

As described above, according to the decontamination device 200B, only the gas containing peracetic acid is released toward the HEPA filters 120 and 130, and thus it is possible to further reduce the likelihood of the HEPA filters 120 and 130 becoming wet, compared to a case where mist containing peracetic acid is released. That is, according to this decontamination device 200A, the HEPA filters 120 and 130 are less likely to become wet, and thus it is possible to reduce the likelihood of the dust collected by the HEPA filters 120 and 130 for particle removal solidifying in a state in which the dust forms a film between fibers and the pressure loss of the HEPA filters 120 and 130 is increased.

In particular, in the decontamination device 200B, the decontamination agent is not heated and peracetic acid gas is released. Therefore, according to the decontamination device 200B, the difference between the temperature inside the safety cabinet 100 and the room temperature does not increase due to the decontamination agent being heated, and dew condensation is less likely to occur in the safety cabinet 100.

### 4. Variations

Although Embodiments 1 to 3 have been described above, the present invention is not limited to Embodiments 1 to 3 described above.

Hereinafter, variations will be described. However, the following variations can be combined as appropriate.

(4-1)
Air filters to be decontaminated are the HEPA filters 120 and 130 in Embodiments 1 to 3 described above. However, air filters to be decontaminated are not limited thereto. Air filters to be decontaminated may be medium efficiency penetration air filters and ULPA filters, for example.

(4-2)
Also, the container that holds an air filter to be decontaminated is the safety cabinet 100 in Embodiments 1 to 3 described above. However, the container that holds an air filter to be decontaminated is not limited thereto. The container that holds an air filter to be decontaminated may be any container as long as it can accommodate the air filter, which is to be decontaminated, inside thereof, for example. Also, the container that holds an air filter to be decontaminated may be sealed or semi-sealed. That is, the degree of sealing of the container may be to a degree such that there is no extreme decrease in the concentration of peracetic acid gas due to leakage of peracetic acid gas. The container may be an isolator device, an incubator, a centrifugal separator, a pass box, a storage cabinet, an air conditioner, or a duct, for example.

(4-3)
Also, it is preferable that the peracetic acid-based decontamination agent is not heated in Embodiments 1 to 3 described above. Furthermore, it is preferable that the peracetic acid-based decontamination agent does not come into contact with a heating element. In a method in which the peracetic acid-based decontamination agent is not heated, when the temperature of a decontamination environment is low, the amount of gas generated is extremely reduced. Therefore, the temperature of the decontamination environment is preferably 10°C or higher, and more preferably 15°C or higher. When the temperature is maintained, the temperature inside the container to be decontaminated and the temperature inside the space (a room or the like) in which the container is disposed are preferably kept at substantially the same temperature. On the other hand, it is known that, when the peracetic acid-based decontamination agent is heated to 40°C or higher, the decomposition of peracetic acid is facilitated. That is, by keeping the peracetic acid-based decontamination agent at a temperature less than 40°C, the decomposition of peracetic acid can be suppressed. Also, by keeping the temperature of the peracetic acid-based decontamination agent at about room temperature, it is possible to suppress the likelihood of dew condensation occurring in the vicinity of the chemical agent.

(4-4)
Also, in Embodiments 1 and 2 described above, the decontamination devices 200 and 200A may be configured to stop the release of gaseous peracetic acid when the humidity inside the safety cabinet 100 reaches a predetermined value. That is, the decontamination devices 200 and 200A may be configured to determine whether or not gaseous peracetic acid can be released in accordance with the output of a humidity sensor for detecting the humidity inside the safety cabinet 100. The above-described predetermined value is RH95% or less, for example.

(4-5)
The decontamination device 200A includes the HEPA filter 260A in Embodiment 2 described above. However, the decontamination device 200A may include a medium efficiency penetration air filter or an ULPA filter instead of the HEPA filter 260A, for example. Also, the materials of these filters may be glass, synthetic fibers, cellulose, or the like.

### 5. Experiments

The following experiments were conducted in order to confirm the effects of the present invention. The following describes the content of the experiments and the results of the experiments.

### 5-1. Experiment 1

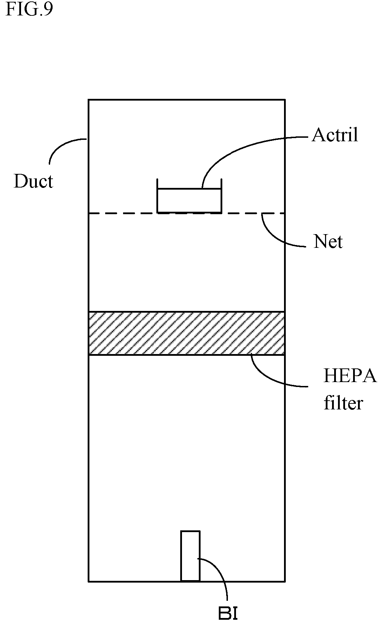

FIG. 9 is a schematic diagram showing an experimental device according to Experiment 1. Referring to FIG. 9, a HEPA filter with a side of 305 mm was installed in a duct with an opening of 275 mm. About 10 ml of Actril (peracetic acid-based disinfectant) manufactured by Mar Cor Purafication Inc. was introduced into a Petri dish with an inner diameter of about 50 mm (having an area of about 20 cm²), the Petri dish was disposed on a net disposed above a HEPA filter, and a BI (Biological Indicator) of the bacteria species *G*. *stearothermophilus* with a bacterial count of 10⁶ was disposed below the HEPA filter. The Petri dish and the BI were left in this state for 12 hours, and then the BI was collected. The collected BI was cultured, and the death of BI was confirmed. Because the BI died out and the completion of decontamination was confirmed, it was estimated that decontamination of the HEPA filter was also completed. Through Experiment 1, it was confirmed that the HEPA filter can be decontaminated by gaseous peracetic acid.

### 5-2. Experiment 2

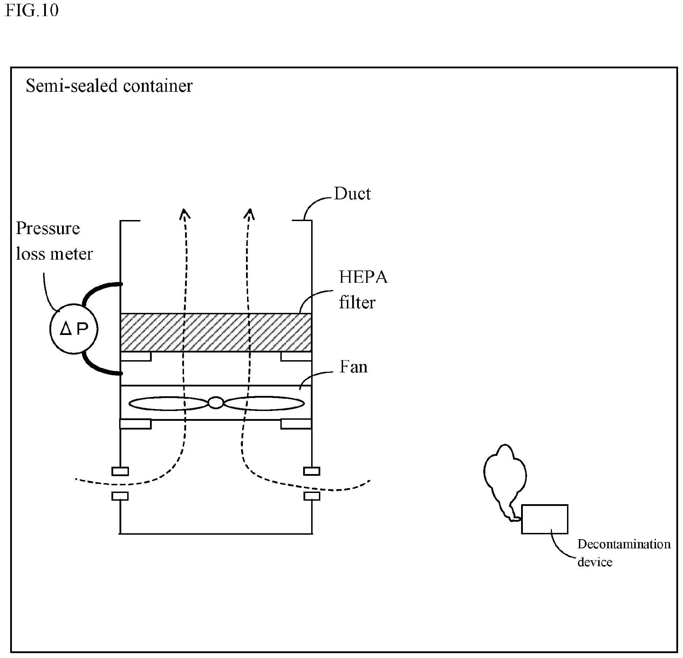

FIG. 10 is a schematic diagram showing an experimental device according to Experiment 2. Referring to FIG. 10, a duct for housing the HEPA filter and the fan, a pressure loss meter for measuring the pressure loss of the HEPA filter, and a decontamination device were disposed in a semi-sealed container. When the decontamination device released mist-like peracetic acid, the pressure loss of the HEPA filter rapidly increased. On the other hand, when the decontamination device released gaseous peracetic acid, the pressure of the HEPA filter did not increase rapidly (the pressure increased by only a few pascals accompanying an increase in humidity). Through Experiment 2, it was confirmed that, as a result of the decontamination device releasing gaseous peracetic acid, it is possible to suppress an increase in the pressure loss of the HEPA filter to be decontaminated.

### 5-3. Experiment 3

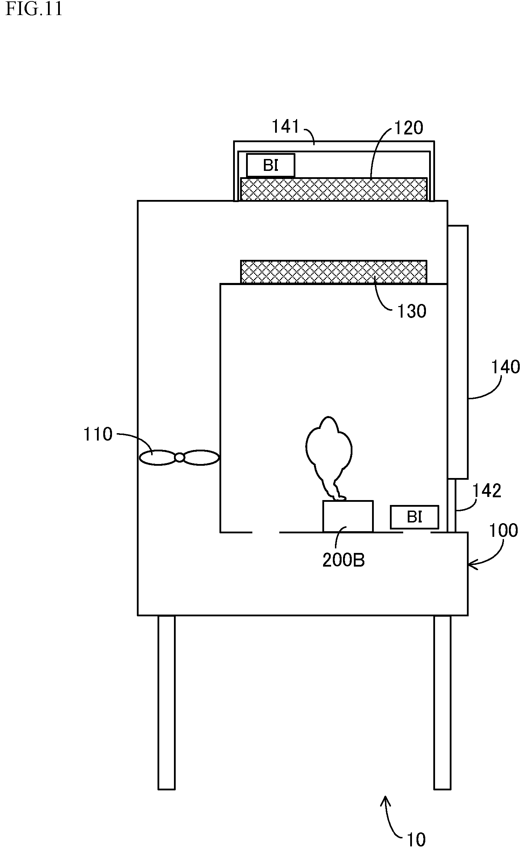

FIG. 11 is a schematic diagram showing an experimental environment in Experiment 3. As shown in FIG. 11, the decontamination device 200B was used in Experiment 3. 1 L of MINNCARE 10% diluted solution was used as the decontamination agent. Three BIs were each disposed in the safety cabinet 100 and above the HEPA filter 120. HMV-091 (with a bacterial count of 10⁶) available from MesaLabs was used as the BIs. During decontamination, the operation in which the fan 110 inside the safety cabinet 100 was operated for 5 minutes and was stopped for 15 minutes was repeated in a state in which the fan 240B of the decontamination device 200B was regularly operated. The decontamination time was five hours from the start of decontamination. The collected BIs were cultured, and the death of all of the BIs was confirmed. Because the BIs died out and the completion of decontamination was confirmed, it was estimated that decontamination of the HEPA filters 120 and 130 was also completed.

### List of Reference Numerals

10 System
100 Safety cabinet
110, 240, 240A, 240B Fan (blower)
120, 130 HEPA filter
260A Mist adsorption filter
140 Shutter
141, 142 Seal member
200, 200A, 200B Decontamination device
210, 210A, 210B Housing
220 Tray
230 Porous member
250A Mist generation device (mist generator)
O1, O2, 01A, O2A Opening portion
S1 Workspace

## Claims

1. A decontamination device (200) configured to decontaminate an air filter (120, 130) for particle removal,
the decontamination device (200) being configured to release gas containing peracetic acid such that the gas reaches the air filter,
the decontamination device (200) is configured not to release mist containing peracetic acid,
the decontamination device (200) comprising
a porous member (230) impregnated with a chemical solution containing peracetic acid;
a housing (210) provided with a fluid flow channel therein and an opening portion (O1A) formed on the downstream side of the fluid flow channel; and
a blower (240) located at the flow channel,
wherein the porous member (230) is disposed at the flow channel.

2. The decontamination device (200) according to claim 1,
wherein the gas is generated without heating a chemical agent containing peracetic acid.

3. A decontamination device (200) configured to decontaminate an air filter (120,130) for particle removal,
the decontamination device (200) being configured to release gas containing peracetic acid such that the gas reaches the air filter,
the decontamination device (200) is configured not to release mist containing peracetic acid,
the decontamination device (200) comprising
a storage body (220) configured to store a chemical solution containing peracetic acid; and
a blower (240) configured to blow air to the gas generated though evaporation of peracetic acid stored in the storage body (220);
wherein the gas to which the air has been blown by the blower (240) reaches the air filter (120, 130).

4. A decontamination device (200) configured to decontaminate an air filter (120, 130) for particle removal,
the decontamination device (200) being configured to release gas containing peracetic acid such that the gas reaches the air filter,
the decontamination device (200) is configured not to release mist containing peracetic acid,
the decontamination device (200) comprising
a housing (210A) provided with a fluid flow channel therein and an opening portion formed on the downstream side of the fluid flow channel;
a mist generator (250A) that is located at the flow channel and is configured to generate mist containing peracetic acid;
a mist adsorption filter (260A) that is located at the downstream side of the flow channel relative to the mist generator (250A) and is configured to adsorb the mist; and
a blower located at the flow channel.

5. A system comprising:
the decontamination device (200) of any one of claims 1 to 4, and
a container configured to hold the air filter for particle removal; wherein
the decontamination device (200) is configured to release gas containing peracetic acid into the container such that the gas reaches the air filter (120, 130) and not release mist containing peracetic acid into the container.

## Patentansprüche

1. Eine Dekontaminationsvorrichtung (200), konfiguriert zum Dekontaminieren eines Luftfilters (120, 130) zum Entfernen von Partikeln,
wobei die Dekontaminationsvorrichtung (200) so konfiguriert ist, dass sie Peroxyessigsäure enthaltendes Gas freisetzt, sodass das Gas den Luftfilter erreicht,
wobei die Dekontaminationsvorrichtung (200) so konfiguriert ist, dass sie keinen Peroxyessigsäure enthaltenden Nebel freisetzt,
die Dekontaminationsvorrichtung (200) umfassend:
ein poröses Element (230), das mit einer Peroxyessigsäure enthaltenden chemischen Lösung imprägniert ist;
ein Gehäuse (210), das mit einem Fluidströmungskanal darin und einem Öffnungsabschnitt (O1A) versehen ist, der auf der stromabwärtigen Seite des Fluidströmungskanals ausgebildet ist; und
ein Gebläse (240), das am Strömungskanal angeordnet ist,
wobei das poröse Element (230) am Strömungskanal angeordnet ist.

2. Die Dekontaminationsvorrichtung (200) gemäß Anspruch 1,
wobei das Gas ohne Erhitzen eines Peroxyessigsäure enthaltenden chemischen Mittels erzeugt wird.

3. Dekontaminationsvorrichtung (200), konfiguriert zum Dekontaminieren eines Luftfilters (120, 130) zum Entfernen von Partikeln,
wobei die Dekontaminationsvorrichtung (200) so konfiguriert ist, dass sie Peroxyessigsäure enthaltendes Gas freisetzt, sodass das Gas den Luftfilter erreicht,
wobei die Dekontaminationsvorrichtung (200) so konfiguriert ist, dass sie keinen Peroxyessigsäure enthaltenden Nebel freisetzt,
die Dekontaminationsvorrichtung (200) umfassend:
einen Speicherkörper (220), der zum Speichern einer Peroxyessigsäure enthaltenden chemischen Lösung konfiguriert ist; und
ein Gebläse (240), das so konfiguriert ist, dass es Luft zu dem Gas bläst, das durch Verdampfen der im Speicherkörper (220) gespeicherten Peroxyessigsäure erzeugt wird;
wobei das Gas, zu dem die Luft durch das Gebläse (240) geblasen wurde, den Luftfilter (120, 130) erreicht.

4. Dekontaminationsvorrichtung (200), konfiguriert zum Dekontaminieren eines Luftfilters (120, 130) zum Entfernen von Partikeln,
wobei die Dekontaminationsvorrichtung (200) so konfiguriert ist, dass sie Peroxyessigsäure enthaltendes Gas freisetzt, sodass das Gas den Luftfilter erreicht,
wobei die Dekontaminationsvorrichtung (200) so konfiguriert ist, dass sie keinen Peroxyessigsäure enthaltenden Nebel freisetzt,
die Dekontaminationsvorrichtung (200) umfassend:
ein Gehäuse (210A) mit einem darin vorgesehenen Fluidströmungskanal und einem Öffnungsabschnitt, der auf der stromabwärtigen Seite des Fluidströmungskanals ausgebildet ist;
einen Nebelgenerator (250A), der am Strömungskanal angeordnet ist und so konfiguriert ist, dass er Peroxyessigsäure enthaltenden Nebel erzeugt;
einen Nebeladsorptionsfilter (260A), der sich in Bezug auf den Nebelgenerator (250A) auf der stromabwärts gelegenen Seite des Strömungskanals befindet und so konfiguriert ist, dass er den Nebel adsorbiert; und
ein Gebläse, das am Strömungskanal angeordnet ist.

5. Ein System, das Folgendes umfasst:
die Dekontaminationsvorrichtung (200) nach einem der Ansprüche 1 bis 4, und
ein Behälter, der so konfiguriert ist, dass er den Luftfilter zur Partikelentfernung aufnehmen kann; wobei
die Dekontaminationsvorrichtung (200) so konfiguriert ist, dass sie Peroxyessigsäure enthaltendes Gas in den Behälter abgibt, sodass das Gas den Luftfilter (120, 130) erreicht, und keinen Peroxyessigsäure enthaltenden Nebel in den Behälter abgibt.

## Revendications

1. Dispositif de décontamination (200) configuré pour décontaminer un filtre à air (120, 130) pour l'élimination des particules,
le dispositif de décontamination (200) étant configuré pour libérer un gaz contenant de l'acide peracétique de sorte que le gaz atteigne le filtre à air,
le dispositif de décontamination (200) est configuré pour ne pas libérer de brouillard contenant de l'acide peracétique,
le dispositif de décontamination (200) comprenant
un élément poreux (230) imprégné d'une solution chimique contenant de l'acide peracétique ;
un boîtier (210) muni d'un canal d'écoulement de fluide et d'une partie ouverte (O1A) formée du côté aval du canal d'écoulement de fluide ; et
un souffleur (240) situé au niveau du canal d'écoulement,
dans lequel l'élément poreux (230) est disposé au niveau du canal d'écoulement.

2. Dispositif de décontamination (200) selon la revendication 1,
dans lequel le gaz est généré sans chauffage d'un agent chimique contenant de l'acide peracétique.

3. Dispositif de décontamination (200) configuré pour décontaminer un filtre à air (120, 130) pour l'élimination des particules,
le dispositif de décontamination (200) étant configuré pour libérer un gaz contenant de l'acide peracétique de sorte que le gaz atteigne le filtre à air,
le dispositif de décontamination (200) est configuré pour ne pas libérer de brouillard contenant de l'acide peracétique,
le dispositif de décontamination (200) comprenant
un corps de stockage (220) configuré pour stocker une solution chimique contenant de l'acide peracétique ; et
un souffleur (240) configuré pour souffler de l'air sur le gaz généré par l'évaporation de l'acide peracétique stocké dans le corps de stockage (220) ;
dans lequel le gaz sur lequel de l'air a été soufflé par le souffleur (240) atteint le filtre à air (120, 130).

4. Dispositif de décontamination (200) configuré pour décontaminer un filtre à air (120, 130) pour l'élimination des particules,
le dispositif de décontamination (200) étant configuré pour libérer un gaz contenant de l'acide peracétique de sorte que le gaz atteigne le filtre à air,
le dispositif de décontamination (200) est configuré pour ne pas libérer de brouillard contenant de l'acide peracétique,
le dispositif de décontamination (200) comprenant
un boîtier (210A) muni d'un canal d'écoulement de fluide et d'une partie d'ouverture formée du côté aval du canal d'écoulement de fluide ;
un générateur de brouillard (250A) situé au niveau du canal d'écoulement et configuré pour générer un brouillard contenant de l'acide peracétique ;
un filtre d'adsorption de brouillard (260A) situé du côté aval du canal d'écoulement par rapport au générateur de brouillard (250A) et configuré pour adsorber le brouillard ; et
un souffleur situé au niveau du canal d'écoulement.

5. Système comprenant :
le dispositif de décontamination (200) selon l'une quelconque des revendications 1 à 4, et
un conteneur configuré pour contenir le filtre à air pour l'élimination des particules ; dans lequel
le dispositif de décontamination (200) est configuré pour libérer du gaz contenant de l'acide peracétique dans le conteneur de sorte que le gaz atteigne le filtre à air (120, 130) et ne libère pas de brouillard contenant de l'acide peracétique dans le conteneur.
